# EUROPEAN PATENT SPECIFICATION

(11) **EP 3 351 932 B1**
(45) Date of publication and mention of the grant of the patent: **30.09.2020**
(21) Application number: 16845891.7
(22) Date of filing: 30.08.2016
(51) Int. Cl.: C12M 1/00, C12M 1/34, G01N 33/483

(54) **ELECTROCHEMICAL MEASUREMENT DEVICE AND ELECTROCHEMICAL MEASUREMENT SYSTEM**
ELEKTROCHEMISCHE MESSVORRICHTUNG UND ELEKTROCHEMISCHES MESSSYSTEM
DISPOSITIF ET SYSTÈME DE MESURE ÉLECTROCHIMIQUE

(30) Priority: 18.09.2015 JP 2015184553
(43) Date of publication of application: 25.07.2018
(73) Proprietor: Panasonic Intellectual Property Management Co., Ltd., Osaka-shi, Osaka 540-6207 (JP)
(72) Inventor: SHUNORI, Atsushi, Oska-shi, Osaka 540-6207 (JP); YASUMI, Masahiro, Oska-shi, Osaka 540-6207 (JP)
(74) Representative: Grünecker Patent- und Rechtsanwälte PartG mbB
(86) International application number: PCT/JP2016/003944
(87) International publication number: WO 2017/047013

(56) References cited:
- EP-A1- 1 504 252
- EP-A1- 2 918 999
- WO-A1-2014/073195
- WO-A1-2016/092803
- JP-A- H06 148 020
- JP-A- H07 286 929
- JP-A- 2000 180 290
- JP-A- 2003 001 214
- JP-A- 2007 132 837
- JP-A- 2008 064 661
- JP-A- 2010 121 948
- JP-A- 2015 194 357
- WU ET AL: "Microfluidic chip integrated with amperometric detector array for in situ estimating oxygen consumption characteristics of single bovine embryos", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER BV, NL, vol. 125, no. 2, 27 July 2007 (2007-07-27) , pages 680-687, XP022296132, ISSN: 0925-4005, DOI: 10.1016/J.SNB.2007.03.017

## Description

### TECHNICAL FIELD

The present disclosure relates to an electrochemical measurement device and an electrochemical measurement system used in examinations and analyses of activity states of biological specimens, such as fertilized ova, including cells and tissue.

### BACKGROUND ART

Biological specimens, fertilized egg, including cells and tissue are active while transporting various materials between insides and outsides of the specimens. For example, a fertilized egg breathes oxygen form its ambient to take it into its cell, and divides in its follicle while consuming the thus-taken oxygen. As means of measuring activity states of such a tissue-derived biomaterial, methods have been known which are carried out to electrically measure variations in physicochemical states that occur around the tissue-derived biomaterial. These methods have been used as procedures of performing pharmacological tests of a new-drug candidate compound by using a model cell, and of measuring the activity of a fertilized egg.

Conventional measurement devices are each provided with a well for accommodating biological specimens. The well is configured with a lower plate and through-holes formed in an upper plate. In the well, an electrode is formed on the upper surface of the lower plate. Moreover, an electrical contact pad is formed on the lower surface of the lower plate. The electrode is electrically connected to the electrical contact pad through a conductive via of the lower plate. Each of the conventional measurement devices performs measurement as follows: An electric current flowing through the electrode disposed on the lower plate, passes through the conductive via to reach the electrical contact pad disposed on the lower surface of the lower plate, thereby allowing the current to be measured to obtain its current value.

PTL 1 is known, for example, as information on conventional technologies related to the present disclosure.

### CITATION LIST

### PATENT LITERATURE

PTL 1: Japanese Patent Laid-Open Publication No. 2008-534965

JP 2007/132837 relates to a cell electrophysiological sensor and its manufacturing method. JP 2008/064661 relates to an apparatus and a method for measuring oxygen. EP 2 918 999 relates to an inspection device for biologically derived material. Ching-Chou Wu et al published in Sensors and Actuators B 125 (2007) 680-687 is about "Microfluidic chip integrated with amperometric detector array for *in situ* estimating oxygen consumption characteristics of single bovine embryos".

EP 1 504 252 relates to a test strip for blood glucose sensing and a method for manufacturing same. JP 2003/001214 relates to a method for detecting defective bonding of impervious sheet and a detector thereof. JP 2000/180290 relates to a water leakage sensor and a relay control circuit board provided therewith.

### SUMMARY

An electrochemical measurement device according to the present invention is defined in claim 1. An electrochemical measurement system according to the invention is defined in claim 14. Further advantageous embodiments are defined in dependent claims.

The electrochemical measurement device and the electrochemical measurement system according to the present disclosure can detect a leakage of the measuring liquid.

### BRIEF DESCRIPTION OF DRAWINGS

FIG. 1 is a perspective view of an electrochemical measurement device according to an exemplary embodiment.
FIG. 2 is a cross-sectional view of the electrochemical measurement device according to the embodiment.
FIG. 3 is a top view of a part of the electrochemical measurement device according to the embodiment.
FIG. 4 is a schematic view of an electrochemical measurement system according to an embodiment.
FIG. 5 is a cross-sectional view of the electrochemical measurement device according to the embodiment for illustrating an operation of the electrochemical measurement device.
FIG. 6 is a cross-sectional view of Modification 1 of the electrochemical measurement device according to the embodiment.
FIG. 7 is a top view of a part of Modification 1 of the electrochemical measurement device according to the embodiment.
FIG. 8 is a top view of a part of Modification 2 of the electrochemical measurement device according to the embodiment.
FIG. 9 is a top view of a part of Modification 3 of the electrochemical measurement device according to the embodiment.
FIG. 10 is a cross-sectional view of Modification 4 of the electrochemical measurement device according to the embodiment.
FIG. 11 is a perspective view of Modification 5 of the electrochemical measurement device according to the embodiment.
FIG. 12 is a cross-sectional view of Modification 5 of the electrochemical measurement device according to the embodiment.
FIG. 13 is a top view of a part of Modification 5 of the electrochemical measurement device according to the embodiment.
FIG. 14 is a schematic view of Modification 6 of the electrochemical measurement system according to the embodiment.
FIG. 15 is a top view of a part of Modification 6 of the electrochemical measurement device according to the embodiment.

### DETAIL DESCRIPTION OF EXEMPLARY EMBODIMENTS

In the conventional measurement device described above, the well is composed of the upper plate and the lower plate. The upper and lower plates are bonded together with adhesive.

In the case where an interstice exists between the upper and lower plates, a measuring liquid injected inside the well may leak through the interstice between the upper and lower plates. In the case where insufficient bonding or poor liquid-sealing provided between the upper and lower plates, the measuring liquid injected inside the well may leak through an interstice between the upper and lower plates. The measuring liquid leaking from the well may contacts other parts including a via-conductor, an electrical contact pad, and a wiring, which are not to contact the liquid.

The contact of the measuring liquid with, e.g. a via-conductor causes an unintended electric current (leakage current) to flow through the measurement device. The leakage current affects electrochemical measurement of a biological specimen, resulting in a decrease in measurement accuracy. For this reason, data from the measurement in the case where the leakage current flows are preferably omitted from the measurement.

The conventional measurement device cannot detect the leakage of the measuring liquid, hence hardly determining whether the leakage current flows or not.

An electrochemical measurement device and an electrochemical measurement system according to an exemplary embodiment of the present disclosure will be described below with reference to accompanying drawings. The embodiment described below merely shows preferable and specific examples of the present disclosure. Numerical values, shapes, materials, constituent elements, and arrangements and connection modes of the constituent elements shown in the following embodiments are mere examples, and therefore are not intended to impose any limitation on the present disclosure. Moreover, of the constituent elements in the following exemplary embodiments, constituent elements not recited in any one of the independent claims which define the most generic concept of the invention are described as optional constituent elements.

Throughout the drawings, figures are schematic ones and their illustrations are not necessarily strictly accurate. Throughout the figures, elements having substantially identical configurations are denoted by the same numerals and symbols, and their duplicate explanations are omitted or simplified.

### Exemplary Embodiment

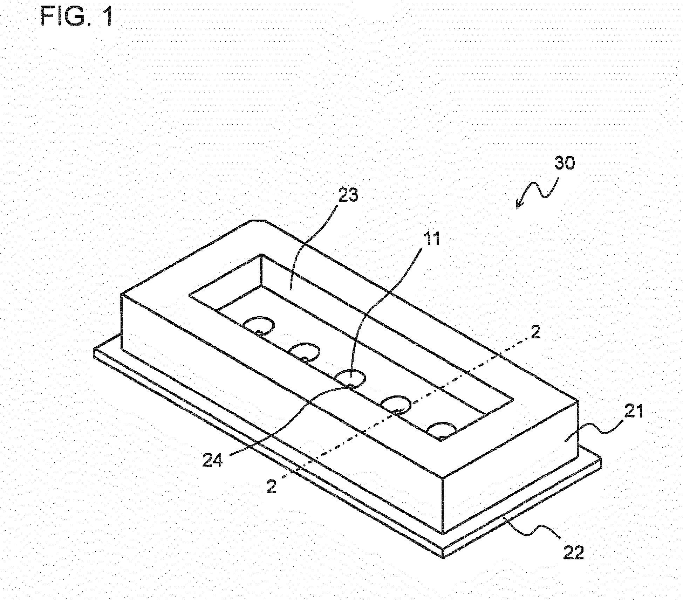

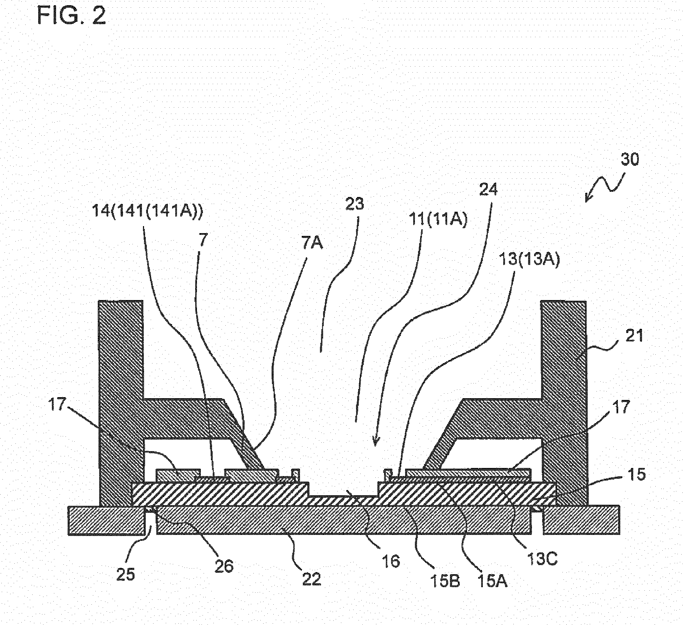

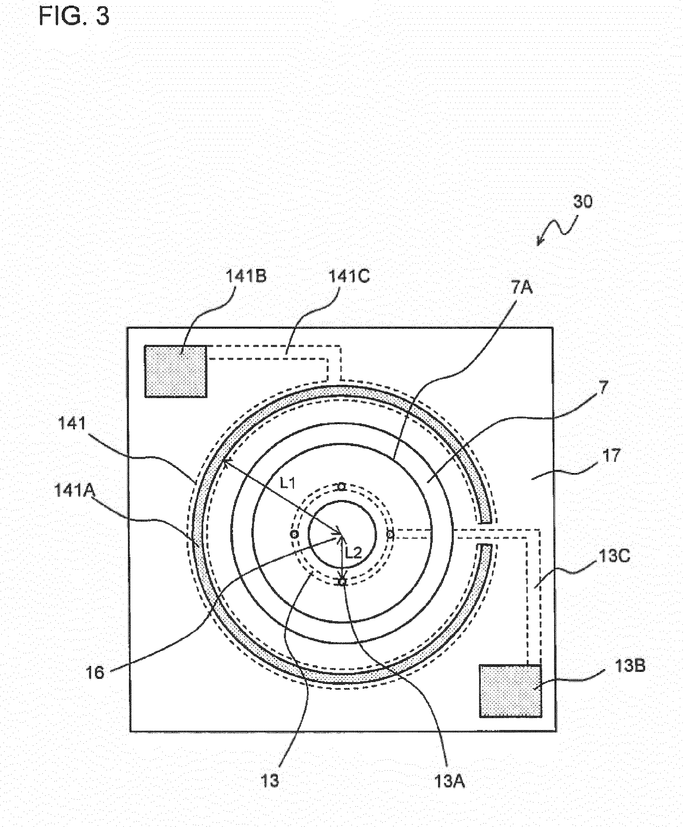

FIG. 1 is a schematic perspective view of electrochemical measurement device 30 according to an exemplary embodiment. FIG. 2 is a schematic cross-sectional view of electrochemical measurement device 30 along line 2-2 line shown in FIG. 1. FIG. 3 is a schematic top view of a bottom of a well, a part of electrochemical measurement device 30.

Electrochemical measurement device 30 is configured to be used to measure activity of biological specimens. The biological specimens include, e.g. cells and tissue of fertilized ova.

Electrochemical measurement device 30 electrochemically measures a specimen introduced in each of wells 11. Electrochemical measurement device 30 includes wall 7 forming each well 11, first working electrode 13 contacting well 11 surrounded by wall 7, and leakage detecting electrode 141 which is leakage detector 14 to detect a leakage of a measuring liquid. Wall 7 has inner wall surface 7A facing well 11. The lower end of the inner wall surface is located between first working electrode 13 and leakage detecting electrode 141 viewing from above electrochemical measurement device 30.

Electrochemical measurement device 30 can detect the leakage current flowing in the measurement device through leakage detecting electrode 141. The leakage current is caused by a leakage of the measuring liquid from well 11. That is, electrochemical measurement device 30 can detect the leakage of the measuring liquid from well 11.

Electrochemical measurement device 30 includes upper plate 21, lower plate 22, and substrate 15. Substrate 15 is disposed between upper plate 21 and lower plate 22. Electrochemical measurement device 30 further includes well 11 into which a measuring liquid containing the biological specimen is introduced.

Upper plate 21 includes reservoir 23 configured to have the measuring liquid put thereto and recess 11A formed in a bottom surface of reservoir 23. Through-hole 24 is formed in the bottom surface of recess 11A. Reservoir 23 is a container to commonly reserve the measuring liquid to be injected to plural wells 11.

Upper plate 21 is made of material, such as glass, resin, silicon, or ceramic. Upper plate 21 may be made of resin material and formed by, e.g. injection molding.

Lower plate 22 is joined with upper plate 21. Substrate 15 is fixed between upper plate 21 and lower plate 22. Through-hole 25 is formed in lower plate 22. Substrate 15 may be fixed to upper plate 21 or lower plate 22 with an adhesive or screws.

Lower plate 22 is made of material, such as glass, resin, silicon, or ceramic. Lower plate 22 may be made of resin material and formed by, e.g. injection molding. Lower plate 22 may be made of the same material as upper plate 21.

Substrate 15 has upper surface 15A and lower surface 15B. First working electrode 13, leakage detecting electrode 141, and placing portion 16 are disposed on upper surface 15A. Substrate 15 is made of material, such as glass, resin, silicon, or ceramic.

The biological specimen, such as a fertilized ovum, is placed on placing portion 16. Placing portion 16 is at a recess formed in the upper surface of substrate 15, for example. The shape of placing portion 16 is optionally determined in accordance with the biological specimen to be measured. For example, placing portion 16 may be a part of upper surface 15A of substrate 15 which is a planar surface.

First working electrode 13 is used in the electrochemical measurement of the measuring liquid.

First working electrode 13 is disposed around placing portion 16. First working electrode 13 has, e.g. a ring shape to surround placing portion 16. First working electrode 13 preferably has a concentric-circle shape about placing portion 16. First working electrode 13 has a thickness of, e.g. 400 nm.

First working electrode 13 is made of metal, such as platinum, gold, or silver. First working electrode 13 may be made of -conductive material, such as carbon or lithium cobalt oxide. The material of first working electrode 13 may be selected in consideration of factors including: a composition of the measuring liquid, a voltage required for the measurement, and influence on biological specimens.

First working electrode 13 having the ring shape may be partly broken, i.e. a ring shape partly opening.

First electrode extracting portion 13B is disposed on a periphery of substrate 15. First electrode extracting portion 13B is connected with first working electrode 13 via wiring 13C. Wall 7 is disposed to overlap a part of wiring 13C.

First working electrode 13 and wiring 13C are covered with insulator layer 17. Insulator layer 17 has a thickness of, e.g. 500 nm. Insulator layer 17 is made of material, such as silicon dioxide, silicon nitride, or organic material.

First working electrode 13 and wiring 13C which are covered with the insulator layer do not directly contact the measuring liquid. Insulator layer 17 can suppress undesired electric-current noises caused by the measuring liquid contacting first working electrode 13 and wiring 13C. The insulator layer can protect first working electrode 13 and wiring 13C. First working electrode 13 includes first electrode-exposed portions 13A that are exposed from insulator layer 17. First electrode-exposed portions 13A can contact the measuring liquid filled in well 11.

Placing portion 16 and first working electrode 13 disposed on substrate 15 are exposed from through-hole 24 of upper plate 21. Parts of first working electrode 13 contact the measuring liquid introduced into well 11.

Recess 11A of upper plate 21 and upper surface 15A of substrate 15 constitutes well 11 of electrochemical measurement device 30. Wall 7 constituting well 11 is a part of upper plate 21 located at a periphery of through-hole 24. Wall 7 surrounds placing portion 16. Portions of wall 7 are preferably located away from placing portion 16 by equal distances over the circumference of wall 7.

Leakage detecting electrode 141 detects a leakage of the measuring liquid from well 11 as a leakage current.

Leakage detecting electrode 141 surrounds the lower end of inner wall surface 7A of wall 7 viewing from above electrochemical measurement device 30. Leakage detecting electrode 141 is disposed over substantially the entire circumference of wall 7. This configuration allows leakage detecting electrode 141 to detect the leakage current regardless of positions on well 7 at which the measuring liquid leaks. Leakage detecting electrode 141 has, e.g. a ring shape with a partly-broken portion, i.e. a ring shape partly opening. Wiring 13C is disposed in the broken portion of the ring shape. This configuration is preferably adopted in cases where, in their manufacturing process, first working electrode 13 and leakage detecting electrode 141 are formed simultaneously in the same process.

A space is disposed above leakage detecting electrode 141.

Leakage detecting electrode 141 is located away from wall 7 by equal distances over the entire leakage detecting electrode.

At the periphery of substrate 15, leakage-detecting electrode extracting portion 141B is disposed on the surface on which leakage detecting electrode 141 is disposed. Leakage-detecting electrode extracting portion 141B is coupled with leakage detecting electrode 141 via wiring 141C. The leakage current flowing into leakage detecting electrode 141 is extracted from leakage-detecting electrode extracting portion 141B. Leakage detecting electrode 141 is disposed between the lower end of inner wall surface 7A and leakage-detecting electrode extracting portion 141B viewing from above.

Leakage detecting electrode 141 and wiring 141C are covered with insulator layer 17. Insulator layer 17 has a thickness of, e.g. 500 nm. Insulator layer 17 is made of material, such as silicon dioxide, silicon nitride, or organic material.

Leakage detecting electrode 141 includes exposed portion 141A that is exposed from insulator layer 17. Exposed portion 141A is preferably disposed along a periphery of wall 7 over substantially the entire circumference of the electrode.

Leakage detecting electrode 141 is located farther from placing portion 16, a reference, than first working electrode 13. That is, distance L1 between the center of placing portion 16 and exposed portion 141A of leakage detecting electrode 141 is larger than distance L2 between the center of placing portion 16 and each of first electrode-exposed portions 13A.

Leakage detecting electrode 141 is made of metal, such as platinum, gold, or silver. Leakage detecting electrode 141 may be made of conductive material, such as carbon or lithium cobalt oxide.

First working electrode 13 and leakage detecting electrode 141 are preferably made of the same material. This configuration allows first working electrode 13 and leakage detecting electrode 141 to be formed simultaneously by the same process.

Coupling part 26 configured to be coupled with an external measurement device is disposed on lower surface 15B of substrate 15. Coupling part 26 is electrically coupled with either first working electrode 13 or leakage detecting electrode 141. This electrical coupling can be achieved by wire bonding or by a via-hole. Coupling part 26 is exposed from through-hole 25 of lower plate 22.

Coupling part 26 is thus disposed above the lower surface of electrochemical measurement device 30. This configuration can detect hat coupling part 26 gets wet with the measuring liquid when the measuring liquid leaks to the outside.

However, the location of coupling part 26 is not necessarily on the lower surface side of electrochemical measurement device 30. Coupling part 26 may be disposed at any location suited for the external measurement device.

As shown in FIGs. 2 and 3, the lower end of wall 7 is disposed between first working electrode 13 and leakage detecting electrode 141 viewing from above electrochemical measurement device 30. Inner wall surface 7A of wall 7 is the surface that faces well 11. That is, well 11 is separated by wall 7 from the outside.

In normal electrochemical measurement device 30, the measuring liquid filling well 11 does not leak to the outside of well 11 due to wall 7 constituting well 11. Here, "normal" electrochemical measurement device 30 is electrochemical measurement device 30 that has no fault in the joining between upper plate 21 and lower plate 22. Accordingly, in normal electrochemical measurement device 30, the measuring liquid does not contact leakage detecting electrode 141. In contrast, in abnormal electrochemical measurement device 30, the measuring liquid may leak to the outside of well 11. Here, "abnormal" electrochemical measurement device 30 is electrochemical measurement device 30 that has an interstice produced between upper plate 21 and lower plate 22. Such an abnormality causes the measuring liquid to contact leakage detecting electrode 141.

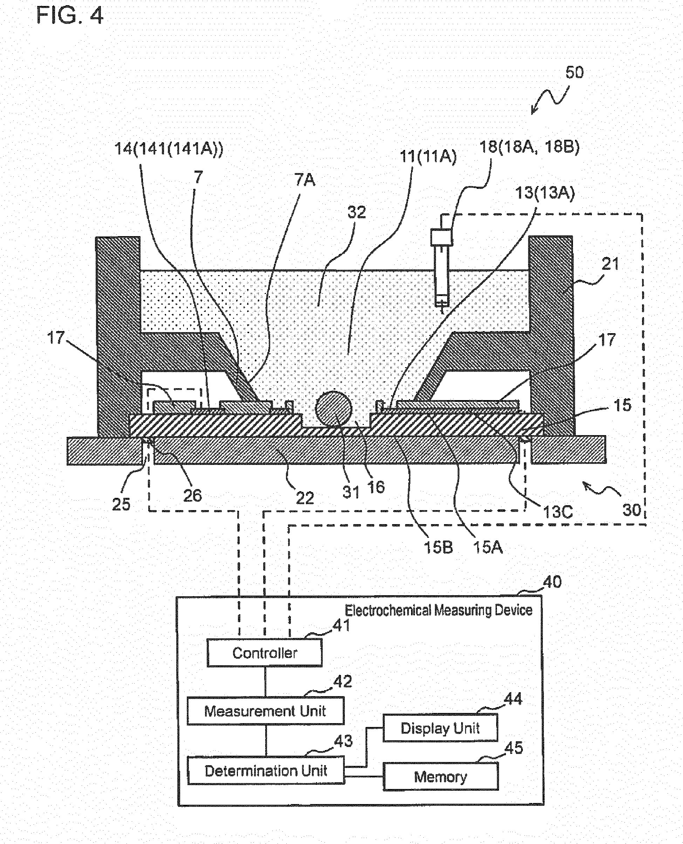

FIG. 4 illustrates electrochemical measurement system 50.

Electrochemical measurement system 50 includes electrochemical measurement device 30 and electrochemical measurement apparatus 40. Electrochemical measurement device 30 is coupled with electrochemical measurement apparatus 40 via coupling part 26.

Measuring liquid 32 is injected into well 11 and reservoir 23 of electrochemical measurement device 30. Biological specimen 31 is placed on placing portion 16. Counter electrode 18 is inserted into measuring liquid 32. Counter electrode 18 has two functions: electrochemically-measuring counter electrode 18A used for electrochemical measurement of biological specimen 31; and leakage-detecting counter electrode 18B used for leakage detection. Electrochemical measurement device 30 measures a current which flows between counter electrode 18 and first working electrode 13.

Counter electrode 18 is made of noble metal, such as platinum, gold, or silver. The material of counter electrode 18 may be selected in consideration of factors including: a composition of the measuring liquid used in the measurement, voltage required for the measurement, and current in the measurement.

In order to accurately detect the electric potential of first working electrode 13, a reference electrode may be disposed at a location allowing the working electrode to contact measuring liquid 32. The reference electrode is made of noble metal, such as platinum, gold, or silver. The material of the reference electrode may be selected in consideration of factors including: a composition of a culture solution used in the measurement, voltage required for the measurement, and current in the measurement.

Counter electrode 18 may be disposed at a location on either substrate 15 or upper plate 21 to allow the counter electrode to contact measuring liquid 32.

Electrochemical measurement apparatus 40 includes controller 41, measurement unit 42, and determination unit 43.

Controller 41 applies an electric potential to first working electrode 13, leakage detecting electrode 141, and counter electrode 18. The electric potential is used for either electrochemical measurement or leakage detection.

For example, a voltage applied between first working electrode 13 and counter electrode 18A causes a current to flow between first working electrode 13 and counter electrode 18A via measuring liquid 32. A voltage applied between leakage detecting electrode 141 and counter electrode 18B, causes a leakage current to flow between leakage detecting electrode 141 and counter electrode 18B when measuring liquid 32 leaks.

Measurement unit 42 measures the current that flows between first working electrode 13 and counter electrode 18A. The value of the current measured by measurement unit 42 allows electrochemical measurement apparatus 40 to measure the state of biological specimen 31.

Measurement unit 42 measures the leakage current that flows between leakage detecting electrode 141 and counter electrode 18B. That is, measurement unit 42 detects whether measuring liquid 32 contacts leakage detecting electrode 141 or not. When the leakage current is measured (i.e., a contact of measuring liquid 32 and leakage detecting electrode 141 is detected), determination unit 43 determines that measuring liquid 32 leaks from well 11.

Controller 41, measurement unit 42, and determination unit 43 are implemented by circuits composed of a sensor, a semiconductor, etc. (i.e. a circuit including: a memory for storing programs, and a processor for executing the programs). Controller 41, measurement unit 42, and determination unit 43 may be configured independently of each other or, alternatively, configured as a single configuration.

Electrochemical measurement apparatus 40 may include display unit 44 for displaying information of the measured current values, results of determinations, etc., and memory 45 for storing the information.

Single counter electrode 18 having the two functions has been described; however, the configuration is not limited to this. For example, electrochemically-measuring counter electrode 18A and leakage-detecting counter electrode 18B may be separately disposed.

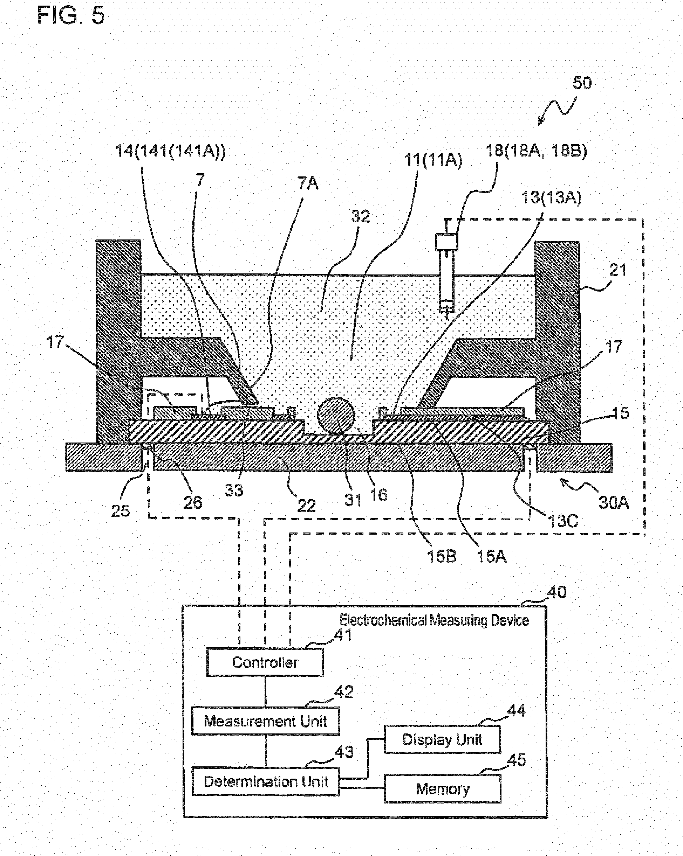

FIG. 5 is a cross-sectional view of electrochemical measurement device 30A for illustrating an operation of electrochemical measurement device 30A.

Electrochemical measurement device 30A is an example of abnormal electrochemical measurement device 30.

Electrochemical measurement device 30A has interstice 33 between wall 7 of upper plate 21 and insulator layer 17 of substrate 15. Measuring liquid 32 leaks through interstice 33 to the outside of well 11. Measuring liquid 32 which leaks contacts leakage detecting electrode 141.

This configuration allows leakage detecting electrode 141 and leakage-detecting counter electrode 18B to be electrically coupled with each other via measuring liquid 32. For this reason, upon applying the voltage between leakage detecting electrode 141 and leakage-detecting counter electrode 18B, a leakage current flows between leakage detecting electrode 141 and leakage-detecting counter electrode 18B. In the case where measuring liquid 32 does not leak from well 11, no leakage current flows between leakage detecting electrode 141 and leakage-detecting counter electrode 18B. Therefore, the measurement of the leakage current allows the device to detect whether measuring liquid 32 has leaked or not.

The leakage current to be measured may be, e.g. a current that flows between first working electrode 13 and leakage detecting electrode 141. In this case, controller 41 applies a voltage between first working electrode 13 and leakage detecting electrode 141. Even the leakage current that flows between first working electrode 13 and leakage detecting electrode 141 allows the device to detect the leakage of measuring liquid 32.

However, the use of first working electrode 13 in measuring the leakage current may facilitate degradation of first working electrode 13. The degradation of first working electrode 13 influences the electrochemical measurement of the specimens. For this reason, the detection of leakage currents is preferably performed by using leakage detecting electrode 141 and counter electrode 18. The use of both leakage detecting electrode 141 and counter electrode 18 prevents first working electrode 13 from degrading due to the measurement of the leakage current.

In electrochemical measurement device 30, plural wells 11 may be disposed. Placing portion 16, first working electrode 13, and leakage detecting electrode 141 are provided in each of wells 11. Counter electrode 18 is disposed in each of wells 11 or only one of wells 11. This configuration, for example, allows the leakage of the measuring liquid in first well 11 to be detect by measuring a leakage current that flows between leakage detecting electrode 141 disposed at a periphery of first well 11 and counter electrode 18 disposed in second well 11 different from first well 11.

In electrochemical measurement device 30 having plural wells 11, leakage detecting electrode 141 may detect a current which flows between leakage detecting electrode 141 of first well 11 and first working electrode 13 of second well 11.

### Modification 1

Modification 1 of the electrochemical measurement device according to the embodiment will be described below with reference to accompanying drawings. Elements with the same configurations as those of the embodiment are denoted by the same numerals and symbols, and their explanations are omitted. Differences of elements between the modification and the embodiment will be detailed.

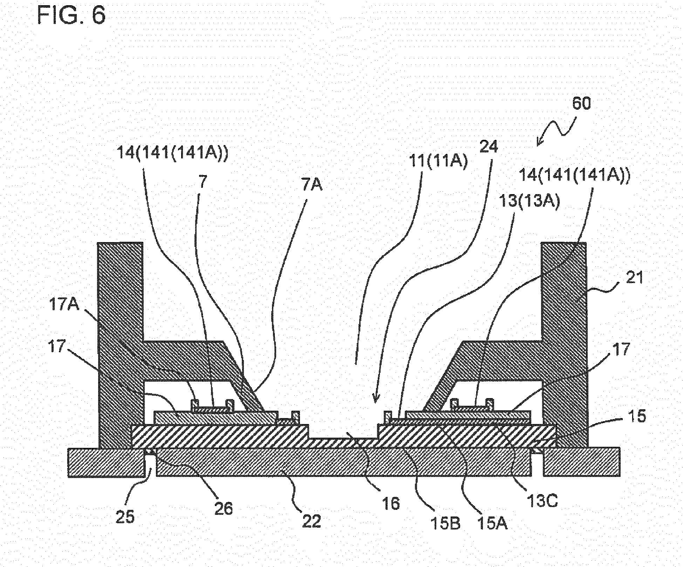

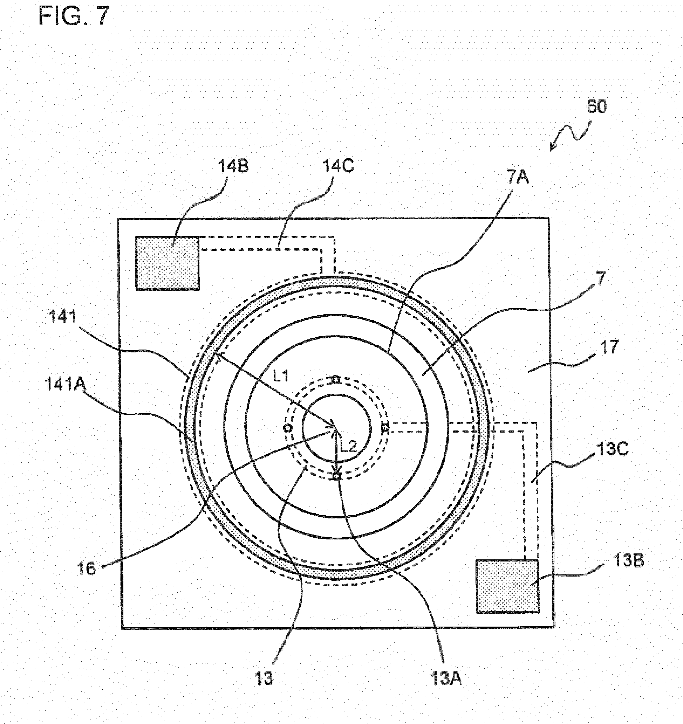

FIG. 6 is a cross-sectional view of electrochemical measurement device 60 of the modification. FIG. 7 is a schematic top view of a bottom portion of a well that is a part of electrochemical measurement device 60 of the modification.

A difference of electrochemical measurement device 60 from electrochemical measurement device 30 shown in FIG. 2 is that leakage detecting electrode 141 is disposed at a different height from that of the first working electrode 13.

Leakage detecting electrode 141 is disposed on the upper surface of insulator layer 17 that is disposed on upper surface 15A of substrate 15.

Electrochemical measurement device 60 is manufactured by the following processes, for example.

First working electrode 13 is formed on upper surface 15A of substrate 15. After that, insulator layer 17 is formed so as to cover first working electrode 13. Next, leakage detecting electrode 141 is formed on the upper surface of insulator layer 17. Finally, insulator layer 17A is formed such that a part of leakage detecting electrode 141 is exposed from insulator layer 17A. Insulator layer 17A covering leakage detecting electrode 141 is not necessarily disposed.

Leakage detecting electrode 141 is thus provided above wiring 13C via insulator layer 17. That is, leakage detecting electrode 141 is disposed also above wiring 13C.

In electrochemical measurement device 60, leakage detecting electrode 141 is disposed along the entire circumference of wall 7.

This configuration allows electrochemical measurement device 60 to reliably detect the leakage of the measuring liquid from well 11 regardless of the direction of the leakage.

### Modification 2

A modification of the electrochemical measurement device according to the embodiment will be described with reference to accompanying drawings. Elements with the same configurations as those of the embodiment are denoted by the same numerals, and their explanations are omitted. A difference of elements between the modification and the embodiment will be detailed.

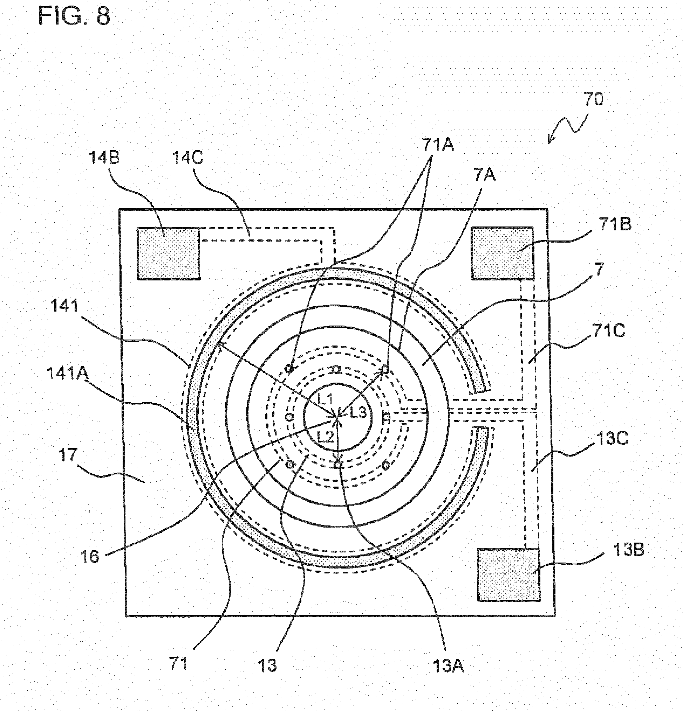

FIG. 8 is a schematic top view of a bottom portion of a well that is a part of electrochemical measurement device 70 of the modification.

A difference of electrochemical measurement device 70 from electrochemical measurement device 30 shown in FIG. 3 is that second working electrode 71 surrounding first working electrode 13.

Second working electrode 71 is disposed around placing portion 16. Second working electrode 71 has, e.g. a ring shape surrounding placing portion 16. Second working electrode 71 preferably has a concentric-circle shape about placing portion 16. Second working electrode 71 has a thickness of, e.g. 400 nm.

Second working electrode 71 and wiring 71C are covered with insulator layer 17.

Second working electrode 71 and wiring 71C which are covered with the insulator layer do not directly contact the measuring liquid. Accordingly, insulator layer 17 can suppress undesired electric-current noises caused by the measuring liquid contacting second working electrode 71 and wiring 71C. In addition, the insulator layer can protect second working electrode 71 and wiring 71C.

Second working electrode 71 includes second electrode-exposed portions 71A that are exposed from insulator layer 17. Second electrode-exposed portions 71A can contact the measuring liquid filling well 11.

Second working electrode 71 is located farther from placing portion 16, a reference, than first working electrode 13. That is, distance L3 between the center of placing portion 16 and each of second electrode-exposed portions 71A is larger than distance L2 between the center of placing portion 16 and each of first electrode-exposed portions 13A.

The lower end of inner wall surface 7A of wall 7 is disposed between second working electrode 71 and leakage detecting electrode 141 viewing from above electrochemical measurement device 70. That is, distance L1 between exposed portion 141A of leakage detecting electrode 141 and the center of placing portion 16 is larger than distance L3 between the center of placing portion 16 and each of second electrode-exposed portions 71A.

Second working electrode 71 having the ring shape may include a partly-broken portion (i.e. a ring shape partly opening). Wiring 13C of first working electrode 13 is disposed in the broken portion of second working electrode 71.

Second working electrode 71 is exposed from through-hole 24 of upper plate 21.

Second working electrode 71 is made of metal, such as platinum, gold, or silver. Second working electrode 71 may be made of conductive material, such as carbon or lithium cobalt oxide.

First working electrode 13, second working electrode 71, and leakage detecting electrode 141 are preferably made of the same material. This configuration allows first working electrode 13, second working electrode 71, and leakage detecting electrode 141 to be formed simultaneously by the same process.

Second working electrode 71 may be disposed so as to overlap wiring 13C of first working electrode 13. In this configuration, an insulator layer is disposed between second working electrode 71 and wiring 13C. Second working electrode 71 having the ring shape is preferably a ring shape with no opening.

Second electrode extracting portion 71B is disposed on a periphery of substrate 15. Second electrode extracting portion 71B is coupled with second working electrode 71 via wiring 71C. Wall 7 is disposed so as to overlap parts of wiring 13C and wiring 71C.

In electrochemical measurement device 70, first working electrode 13 and second working electrode 71 are disposed at different locations with different distances from placing portion 16.

This configuration allows electrochemical measurement device 70 to perform electrochemical measurement of the measuring liquid containing a biological specimen, at the different locations with the different distances from the biological specimen. Accordingly, electrochemical measurement device 70 can measure variations in the measuring liquid with respect to the distance from the biological specimen. Electrochemical measurement device 70 has advantages, over electrochemical measurement device 30 without second working electrode 71, that it can more accurately detect the state of the biological specimen.

### Modification 3

An electrochemical measurement device of a modification according to the embodiment will be described below with reference to accompanying drawings. Elements with the same configurations as those of the embodiment are denoted by the same numerals, and their explanations are omitted. Differences of elements between the modification and the embodiment will be detailed.

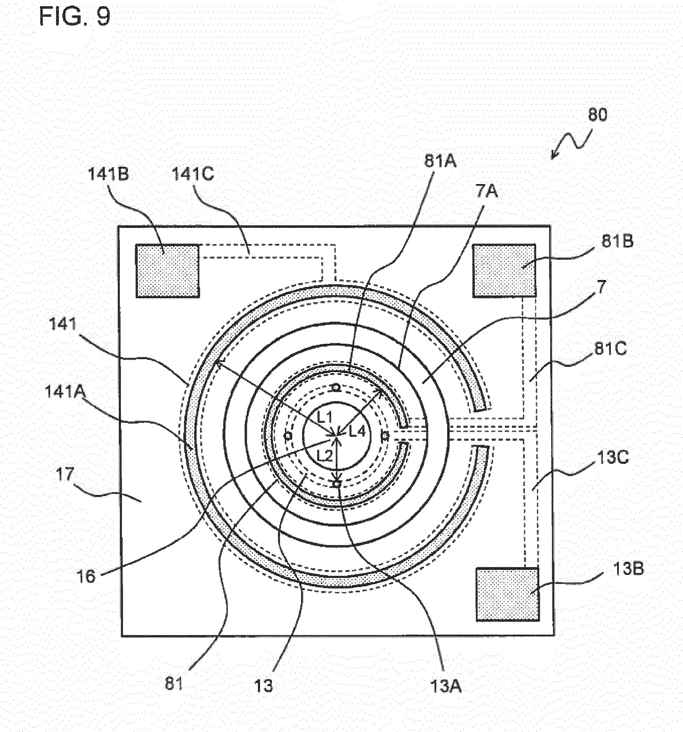

FIG. 9 is a schematic top view of a bottom portion of a well that is a part of electrochemical measurement device 80 of the modification.

A difference of electrochemical measurement device 80 from electrochemical measurement device 30 shown in FIG. 3 is that leakage-detecting counter electrode 81 is disposed so as to surround first working electrode 13.

Counter electrode 81 is disposed around placing portion 16. Counter electrode 81 has, e.g. a ring shape surrounding placing portion 16. Counter electrode 81 preferably has a concentric-circle shape about placing portion 16. Counter electrode 81 has a thickness of, e.g. 400 nm.

Counter electrode 81 having the ring shape includes a partly-broken portion (i.e. a ring shape partly opening). Wiring 13C of first working electrode 13 is disposed in the broken portion of counter electrode 81.

Counter electrode 81 may be disposed so as to overlap wiring 13C of first working electrode 13. In this configuration, an insulator layer is disposed between counter electrode 81 and wiring 13C. Counter electrode 81 preferably has a ring shape with no opening.

Counter electrode extracting portion 81B is disposed on a periphery of substrate 15. Counter-electrode extracting portion 81B is coupled with counter electrode 81 via wiring 81C. Wall 7 is disposed so as to overlap parts of wiring 13C and wiring 81C.

Counter electrode 81 and wiring 81C are covered with insulator layer 17. Counter electrode 81 and wiring 81C which are covered with insulator layer 17 do not directly contact the measuring liquid. Accordingly, insulator layer 17 can suppress undesired electric-current noises caused by the measuring liquid contacting counter electrode 81 and wiring 81C. In addition, the insulator layer can protect counter electrode 81 and wiring 81C.

Counter electrode 81 includes counter-electrode-exposed portion 81A that is exposed from insulator layer 17. Counter-electrode-exposed portion 81A can contact the measuring liquid filling well 11.

Counter electrode 81 is located farther from placing portion 16, a reference, than first working electrode 13. That is, distance L4 between counter-electrode-exposed portion 81A and the center of placing portion 16 is larger than distance L2 between the center of placing portion 16 and each of first electrode-exposed portions 13A.

Counter electrode 81 is exposed from through-hole 24 of upper plate 21.

Inner wall surface 7A of wall 7 is disposed between counter electrode 81 and leakage detecting electrode 141 viewing from above electrochemical measurement device 80. That is, distance L1 between exposed portion 141A of leakage detecting electrode 141 and the center of placing portion 16 is larger than distance L4 between counter-electrode-exposed portion 81A and the center of placing portion 16.

Counter electrode 81 is made of metal, such as platinum, gold, or silver. Counter electrode 81 may be made of conductive material, such as carbon or lithium cobalt oxide.

First working electrode 13, counter electrode 81, and leakage detecting electrode 141 are preferably made of the same material. This configuration allows first working electrode 13, counter electrode 81, and leakage detecting electrode 141 to be formed simultaneously by the same process.

Counter electrode 81 may be used also as a counter electrode for electrochemical measurement.

Electrochemical measurement device 80 thus includes counter electrode 81 on the upper surface of substrate 15. Accordingly, there is no need for separately disposing counter electrode 18 to perform electrochemical measurement.

This configuration reduces steps of operations for the electrochemical measurement with electrochemical measurement device 80.

### Modification 4

An electrochemical measurement device of a modification according to the embodiment will be described below with reference to accompanying drawings. Elements with the same configurations as those of the embodiment are denoted by the same numerals, and their explanations are omitted. Differences of elements between the modification and the embodiment will be detailed.

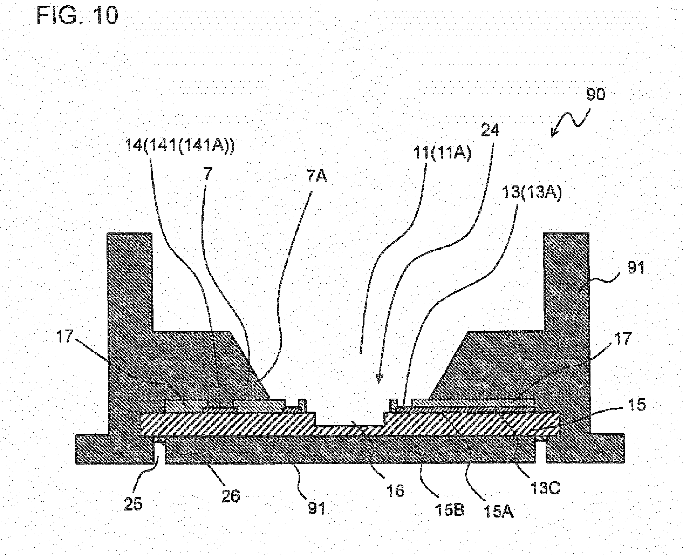

FIG. 10 is a cross-sectional view of electrochemical measurement device 90 of the modification according to the embodiment.

A difference of electrochemical measurement device 90 from electrochemical measurement device 30 shown in FIG. 3 is that leakage detecting electrode 141 is disposed inside wall 7 made of a resin.

Plate 91 of electrochemical measurement device 90 is a frame having substrate 15 buried therein.

Plate 91 is made of a resin and formed by integral molding. Wall 7 is a part of plate 91 forming well 11.

Leakage detecting electrode 141 is disposed inside wall 7. That is, the resin of wall 7 is disposed so as to cover leakage detecting electrode 141. The surface of leakage detecting electrode 141 contacts the resin of wall 7.

In a case where electrochemical measurement device 90 is abnormal, a small interstice may be produced between substrate 15 and plate 91. In addition, a small interstice may be produced between leakage detecting electrode 141 and wall 7 as well. This allows the measuring liquid to move, by a capillary phenomenon, through the small interstices of abnormal electrochemical measurement device 90. Accordingly, the measuring liquid can move to reach leakage detecting electrode 141. This causes electrochemical measurement device 90 to detect the leakage of the measuring liquid.

Electrochemical measurement device 90 is manufactured, for example, as follows.

Substrate 15 having first working electrode 13 and leakage detecting electrode 141 provided thereon is prepared. Next, substrate 15 is placed in a metal mold for plate 91. Then, a resin is injected to fill the metal mold in which substrate 15 is placed. Finally, the injected resin is cooled to solidify plate 91 having substrate 15 buried therein.

This configuration allows large-volume and low-cost manufacturing of electrochemical measurement devices 90.

### Modification 5

An electrochemical measurement device of a modification according to the embodiment will be described below with reference to accompanying drawings. Elements with the same configurations as those of the embodiment are denoted by the same numerals, and their explanations are omitted. Differences of elements between the modification and the embodiment will be detailed below.

A difference of electrochemical measurement device 100 from electrochemical measurement device 30 shown in FIG. 3 is that at least one well of plural wells 11 the device is well 120 which has leakage-detecting counter electrode 121 therein instead of first working electrode 13.

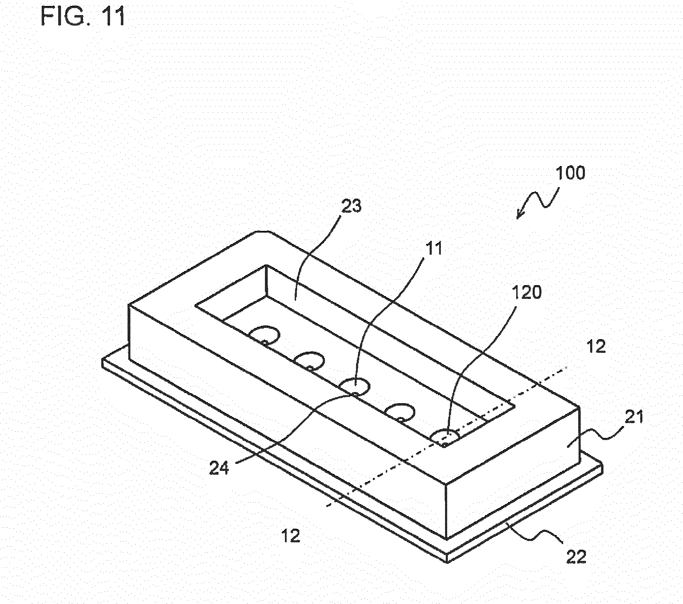

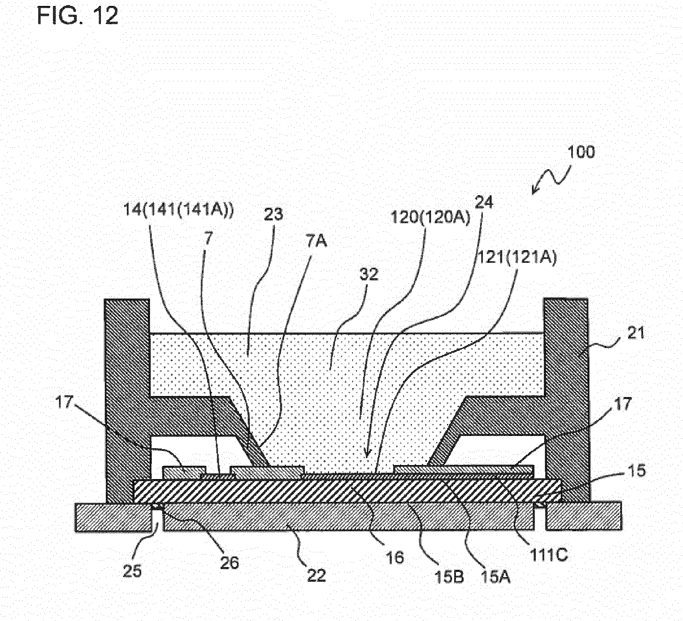

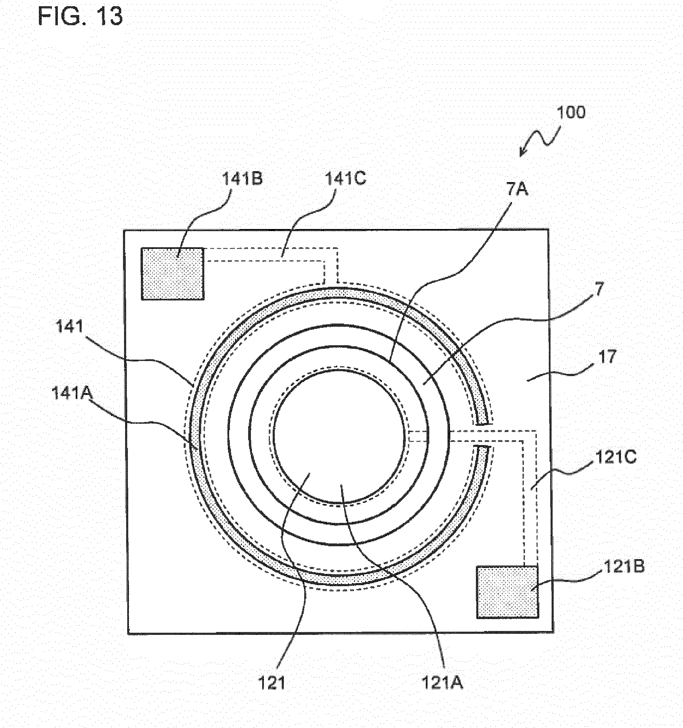

FIG. 11 is a schematic perspective view of electrochemical measurement device 100 of the modification. FIG. 12 is a schematic cross-sectional view of electrochemical measurement device 100 along line 12-12 shown in FIG. 11. FIG. 13 is a schematic top view of a bottom portion of well 120, i.e. a part of electrochemical measurement device 100.

Substrate 15 and wall 7 of upper plate 21 constitute well 120. Counter electrode 121 for detecting leakage is disposed on the bottom surface of well 120.

First working electrode 13, second working electrode 71, and placing portion 16 are not disposed in well 120. That is, biological specimen 31 is not placed in well 20. The electrochemical measurement of a specimen is not performed in well 120.

Only counter electrode 121 for detecting leakage is disposed in well 120, for example. However, counter electrode 121 has a function of a counter electrode for electrochemical measurement as well.

Counter electrode 121 has, e.g. a circular shape. The thickness of counter electrode 121 is, e.g. 400 nm.

Counter-electrode extracting portion 121B is disposed on a periphery of substrate 15. Counter-electrode extracting portion 121B is coupled with counter electrode 121 via wiring 121C. Wall 7 is disposed so as to overlap a part of wiring 121C.

Counter electrode 121 is exposed from through-hole 24 of upper plate 21.

Counter electrode 121 and wiring 121C are covered with insulator layer 17. Counter electrode 121 and wiring 121C covered with insulator layer 17 do not directly contact the measuring liquid. Accordingly, insulator layer 17 can suppress undesired electric-current noises caused by the measuring liquid contacting counter electrode 121 and wiring 121C. In addition, the insulator layer can protect counter electrode 121 and wiring 121C.

Counter electrode 121 includes counter-electrode-exposed portion 121A that is exposed from insulator layer 17. Counter-electrode-exposed portion 121A can contact the measuring liquid filling well 120.

Counter electrode 121 is made of metal, such as platinum, gold, or silver. Counter electrode 121 may be made of conductive material, such as carbon or lithium cobalt oxide.

Leakage detecting electrode 141 is disposed so as to circumferentially surround counter electrode 121.

The lower end of inner wall surface 7A of wall 7 is disposed between counter electrode 121 and leakage detecting electrode 141 viewing from above electrochemical measurement device 100.

With this configuration, a leakage of measuring liquid 32 can be detected in well 120 as well.

Electrochemical measurement device 100 includes reservoir 23 provided above well 11 and well 120.

In electrochemical measurement, measuring liquid 32 is poured to fill well 11 and well 120. Measuring liquid 32 is further poured to fill reservoir 23 of upper plate 21. This configuration electrically couples leakage detecting electrode 141 in well 11 with leakage-detecting counter electrode 121 in well 120 different from well 11 via measuring liquid 32 filling wells 11 and 120 and reservoir 23.

Controller 41 of electrochemical measurement apparatus 40 applies an electric voltage between leakage detecting electrode 141 of well 11 and counter electrode 121 of well 120. In a case where measuring liquid 32 leaks from well 11, a leakage current flows between leakage detecting electrode 141 of well 11 and counter electrode 121 of well 120. By measuring the leakage current, electrochemical measurement apparatus 40 detects the leakage of measuring liquid 32 in well 11.

The first well means well 11 while the second well means well 120.

In this configuration, the leakage of measuring liquid 32 from well 11 can be detected by using counter electrode 121 that is disposed in well 120 different from the well concerned.

Similarly, either first working electrode 13 or second working electrode 71 which is disposed in well 11 is also electrically coupled with counter electrode 121 disposed in well 120 via measuring liquid 32. Accordingly, electrochemical measurement device 100 can performing the electrochemical measurement of a specimen, by applying a voltage between first working electrode 13 of well 11 and counter electrode 121 of well 120.

Controller 41 applies a voltage between leakage detecting electrode 141 in well 120 and counter electrode 121 in well 120. In a case where measuring liquid 32 leaks from well 120, a leakage current flows between leakage detecting electrode 141 in well 120 and counter electrode 121 in well 120. By measuring the leakage current, electrochemical measurement apparatus 40 can detect the leakage of measuring liquid 32 from well 120. The electrochemical measurement device does not necessarily include exposed portion 141A at a periphery of well 120 since electrochemical measurement of specimens is not performed in well 120.

This configuration allows electrochemical measurement device 100 not to be separately equipped with a counter electrode in well 11. This configuration reduces steps of operations for the electrochemical measurement in electrochemical measurement device 100.

The counter electrode may be disposed in well 11 or, alternatively, inserted into the well.

In another example, only counter electrode 121 and a reference electrode are disposed in well 120. Counter electrode 121 may be such that a leakage-detecting counter electrode and an electrochemically-measuring counter electrode are separately disposed.

### Modification 6

An electrochemical measurement device of Modification 6 according to the embodiment will be described below with reference to accompanying drawings. Elements with the same configurations as those of the embodiment are denoted by the same numerals, and their explanations are omitted. Differences of elements between the modified example and the embodiment will be detailed.

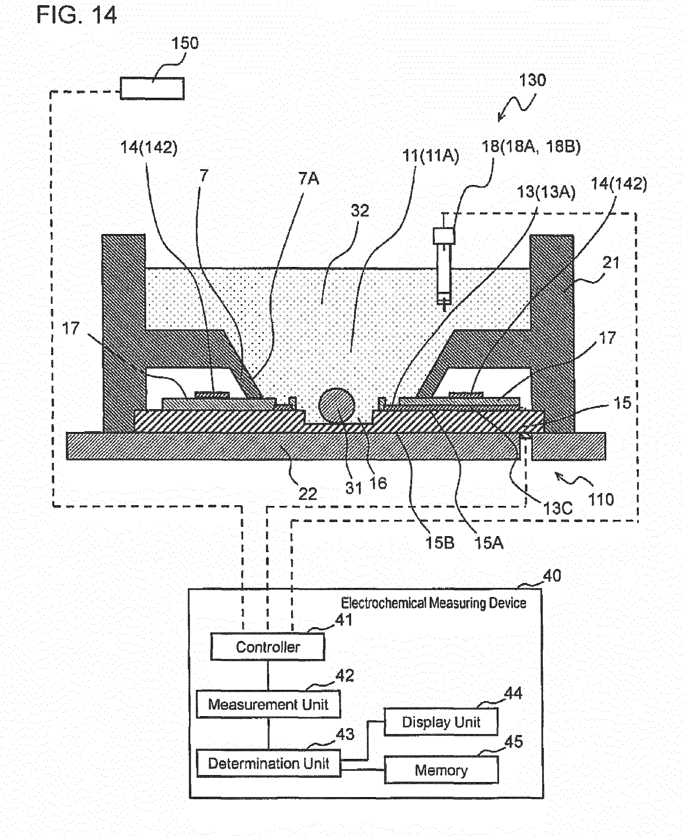

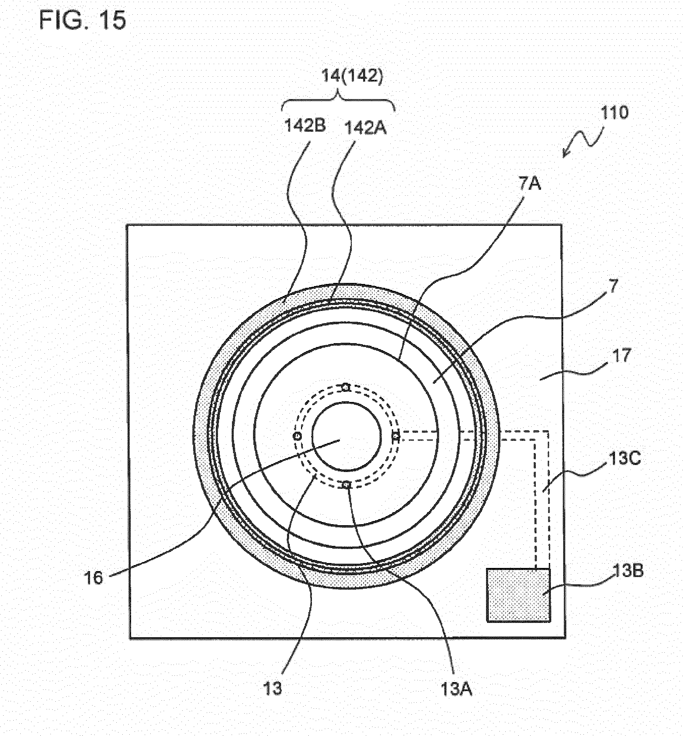

FIG. 14 illustrates electrochemical measurement system 130 including electrochemical measurement device 110 of the modification. Electrochemical measurement system 130 includes electrochemical measurement device 110 and electrochemical measurement apparatus 40. FIG. 15 is a schematic top view of a bottom portion of well 11 that is a part of electrochemical measurement device 110 of the modification.

A difference of electrochemical measurement device 110 from electrochemical measurement device 30 shown in FIG. 2 is that leakage-detecting discoloration portion 142 is used as leakage detector 14.

Upon contacting measuring liquid 32, leakage-detecting discoloration portion 142 has color change. In electrochemical measurement system 130, measurement unit 42 detects the change in color of leakage-detecting discoloration portion 142 caused by measuring liquid 32 leaking from well 11. The change of the color can be detected by a visual recognition or an image recognition performed by image sensor 150 coupled via controller 41. In a case where image sensor 150 is used, for example, the leakage of measuring liquid 32 can be detected by inputting an output signal of image sensor 150 into electrochemical measurement apparatus 40. Determination unit 43 can determine that measuring liquid 32 leaks from well 11 when measurement unit 42 detects a change in color of leakage-detecting discoloration portion 142 through use of image sensor 150. In a case where leakage-detecting discoloration portion 142 is used as leakage detector 14, leakage-detecting discoloration portion 142 does not require a detecting-electrode extracting portion or a wiring.

Leakage-detecting discoloration portion 142 is disposed around wall 7. Leakage-detecting discoloration portion 142 has, e.g. a ring shape so as to surround wall 7 viewing from above electrochemical measurement device 110. Leakage-detecting discoloration portion 142 is preferably concentric with inner wall surface 7A of wall 7.

Leakage-detecting discoloration portion 142 includes, e.g. paper sheet 142B coated with water-soluble coloring agent 142A. Water-soluble coloring agent 142A is applied to paper sheet 142B having the ring shape continuously along a circumferential direction of the ring shape. Water-soluble coloring agent 142A may be applied to paper sheet 142B intermittently along the circumferential direction. Water-soluble coloring agent 142A is arranged at an inner circumference portion of leakage-detecting discoloration portion 142. Upon contacting measuring liquid 32, water-soluble coloring agent 142A dissolves and diffuses into paper sheet 142B, thereby changing the color of paper sheet 142B. Water-soluble coloring agent 142A may be fluorescein or fluorescein sodium. Fluorescein and sodium fluorescein sodium change their color tones upon contacting water, thereby easily recognizing the change in color of leakage-detecting discoloration portion 142. Leakage-detecting discoloration portion 142 may have a structure body in which water-soluble coloring agent 142A is patterned. In a case where such a structure body including water-soluble coloring agent 142A is used, paper sheet 142B is not necessarily used as leakage-detecting discoloration portion 142.

Upper plate 21 is preferably made of material, such as a glass, resin, silicon, or ceramic, that has high optical transparency.

The configurations of first working electrode 13, second working electrode 71, leakage detector 14, and counter electrode 81 which are combined as described above in the embodiment and Modifications 1 to 6 may be combined to have a different combination. The electrochemical measurement device having such a different combination can provide advantageous effects in accordance with each combined configuration. The electrochemical measurement system may include the electrochemical measurement device having such a different combination and electrochemical measurement apparatus 40.

In accordance with the embodiment, the electrodes, such as first working electrode 13, second working electrode 71, leakage detecting electrode 141, and counter electrode 81 have ring shapes. However, first working electrode 13, second working electrode 71, leakage detecting electrode 141, and counter electrode 81 do not necessarily have ring shapes. For example, first working electrode 13, second working electrode 71, leakage detecting electrode 141, and counter electrode 81 may be point electrodes. First working electrode 13, second working electrode 71, leakage detecting electrode 141, and counter electrode 81 may have a polygonal shape or an ellipse shape.

First working electrode 13, second working electrode 71, leakage detecting electrode 141, counter electrode 81, and placing portion 16 may be disposed on lower plate 22.

In the present invention, terms, such as "upper surface," "lower surface," "upper," and "lower", indicating directions indicates relative directions determined by only a relative positional relation of constituent elements of the electrochemical measurement device, and do not indicate absolute directions, such as a vertical direction.

Although the electrochemical measurement devices and electrochemical measurement systems according to one or more aspects have been described based on the aforementioned exemplary embodiment and modified examples, the present disclosure is obviously not limited to such exemplary embodiment and modified examples. Other forms in which various modifications apparent to those skilled in the art are applied to the exemplary embodiment and modifications, or forms structured by combining the structural elements of the exemplary embodiment and modified examples may be included within the scope of the one or plurality of aspects, unless such changes and modifications depart from the scope of the present disclosure.

### INDUSTRIAL APPLICABILITY

An electrochemical measurement device and an electrochemical measurement system according to the present disclosure are useful to examinations and analyses of activity states of biological specimens.

### REFERENCE MARKS IN THE DRAWINGS

- 7: wall
- 7A: inner wall surface
- 11, 120: well
- 13: first working electrode
- 141: leakage detecting electrode
- 142: leakage-detecting discoloration portion
- 15: substrate
- 16: placing portion
- 17, 17A: insulator layer
- 21: upper plate
- 22: lower plate
- 23: reservoir
- 24, 25: through-hole
- 26: coupling part
- 30, 30A, 60, 70, 80, 90, 100, 110: electrochemical measurement device
- 40: electrochemical measurement apparatus
- 41: controller
- 42: measurement unit
- 43: determination unit
- 44: display unit
- 45: memory
- 50, 130: electrochemical measurement system
- 71: second working electrode
- 18, 18A, 18B, 81, 121: counter electrode
- 91: plate
- 150: image sensor

## Claims

1. An electrochemical measurement device configured to electrochemically measure a specimen with using a measuring liquid, the electrochemical measurement device comprising:
a first well (11) configured to have the specimen placed therein and have the measuring liquid injected thereto;
a wall (7) having an inner wall surface (7A) forming the first well (11), the wall (7) surrounding the first well (11);
a first working electrode (13) contacting the first well (11), the first working electrode (13) being configured to have an electric potential applied thereto; and
a leakage detector (14) that detects a leakage of the measuring liquid,
**characterized in that,** the first working electrode (13), the wall (7) and the leakage detector (14) are arranged such that the first working electrode (13) is surrounded by the wall (7) defining the first well (11) and by the leakage detector (14, 141, 142), wherein a lower end of the inner wall surface (7A) is disposed between the first working electrode (13) and the leakage detector (14),
wherein the leakage detector (14) includes a leakage detecting electrode (141) for detecting a leakage current.

2. The electrochemical measurement device according to claim 1, wherein the leakage detecting electrode (141) surrounds the inner wall surface (7A).

3. The electrochemical measurement device according to claim 2, wherein the leakage detecting electrode (141) has a ring shape.

4. The electrochemical measurement device according to claim 3, wherein the leakage detecting electrode (141) has a ring shape partly opening.

5. The electrochemical measurement device according to claim 1, wherein the leakage current flows in the leakage detecting electrode (141), the electrochemical measurement device further comprising
a leakage-detecting electrode extracting portion (141B) disposed on a surface on which the leakage detecting electrode (141) is disposed, the leakage detecting electrode extracting portion (141B) being coupled with the leakage detecting electrode (141) via a wiring (141C), the leakage-detecting electrode extracting portion (141B) being configured to extract the leakage current flowing in the leakage detecting electrode (141), and
wherein the leakage detecting electrode (141) is disposed between the inner wall surface (7A) and the leakage-detecting electrode extracting portion (141B).

6. The electrochemical measurement device according to claim 1,
wherein the wall (17) is made of a resin,
wherein the leakage detecting electrode (141) is disposed in the wall (17), and
wherein a surface of the leakage detecting electrode (141) contacts the resin.

7. The electrochemical measurement device according to claim 1, further comprising
a placing portion (16) disposed in the first well (11), the placing portion (16) being configured to have the specimen placed thereon,
wherein the first working electrode (13) surrounds the placing portion (16).

8. The electrochemical measurement device according to claim 7, further comprising a second working electrode (71) surrounding the first working electrode (13).

9. The electrochemical measurement device according to claim 1, further comprising a counter electrode (18, 18A, 18B, 81, 121) disposed in the first well (11), the counter electrode being configured to have an electric potential applied thereto.

10. The electrochemical measurement device according to claim 1, further comprising:
a second well (11) different from the first well (11);
a counter electrode (18, 18A, 18B, 81, 121) disposed in the second well (11), the counter electrode being configured to have an electric potential used for measurement applied thereto; and
a reservoir (23) disposed above the first well (11) and the second well (11), the reservoir (23) being configured to commonly reserve the measuring liquid to be injected to the first well (11) and the second well (11).

11. An electrochemical measurement device configured to electrochemically measure a specimen with using a measuring liquid, the electrochemical measurement device comprising:
a first well (11) configured to have the specimen placed therein and have the measuring liquid injected thereto;
a wall (7) having an inner wall surface (7A) forming the first well (11), the wall (7) surrounding the first well (11);
a first working electrode (13) contacting the first well (11), the first working electrode (13) being configured to have an electric potential applied thereto; and
a leakage detector (14) that detects a leakage of the measuring liquid,
**characterized in that,** the first working electrode (13), the wall (7) and the leakage detector (14) are arranged such that the first working electrode (13) is surrounded by the wall (7) defining the first well (11) and by the leakage detector (14, 141, 142), wherein a lower end of the inner wall surface (7A) is disposed between the first working electrode (13) and the leakage detector (14),
wherein the leakage detector (14) comprises a leakage-detecting discoloration portion (142) having color changing upon contacting the measuring liquid.

12. The electrochemical measurement device according to claim 11, wherein the leakage-detecting discoloration portion (142) is a paper sheet coated with a water-soluble coloring agent.

13. An electrochemical measurement system configured to electrochemically measure a specimen with using a measuring liquid, the electrochemical measurement system comprising an electrochemical measurement device (30, 30A, 60, 70, 80, 90, 100, 110) according to claim 1 and an electrochemical measurement apparatus,
wherein the electrochemical measurement apparatus includes:
a controller (41) that apples an electric potential to the working electrode (13);
a measurement unit (42) that detects whether the measuring liquid contacts the leakage detector (14) or not; and
a determination unit (43) that, when the measurement unit (42) detects that the measuring liquid contacts the leakage detector (14), determines that the measuring liquid in the first well (11) leaks.

14. The electrochemical measurement system according to claim 13,
wherein, when the leakage detecting electrode (141) detects the leakage current, the determination unit (43) determines that the measuring liquid in the first well (11) leaks.

15. The electrochemical measurement system according to claim 14,
wherein the electrochemical measurement device further includes a counter electrode (18) inserted into the first well (11) so as to contact the measuring liquid, the counter electrode (18) being configured to have an electric potential applied thereto,
wherein the leakage current flows between the counter electrode (18) and the leakage detecting electrode (141), and
wherein the measurement unit (42) measures the leakage current flowing between the counter electrode (18) and the leakage detecting electrode (141).

16. The electrochemical measurement system according to claim 14,
wherein the electrochemical measurement device further includes a counter electrode (18) disposed in the first well (11), the counter electrode (18) being configured to have an electric potential applied thereto,
wherein the leakage current flows between the counter electrode (18) and the leakage detecting electrode (141), and
wherein the measurement unit (42) measures the leakage current flowing between the counter electrode (18) and the leakage detecting electrode (141).

17. The electrochemical measurement system according to claim 14,
wherein the electrochemical measurement device further includes:
a second well (11) different from the first well (11);
a counter electrode (18) disposed in the second well (11), the counter electrode (18) being configured to have an electric potential; and
a reservoir (23) disposed above the first well (11) and the second well (11), the reservoir (23) being configured to commonly reserve the measuring liquid to be injected to the first well (11) and the second well (11), and
wherein the measurement unit (42) measures a current flowing between the counter electrode (18) and the leakage detecting electrode (141).

18. The electrochemical measurement system according to claim 14,
wherein the leakage current flows between the working electrode (13) and the leakage detecting electrode (141), and
wherein the measurement unit (42) measures the leakage current flowing between the working electrode (13) and the leakage detecting electrode (141).

19. An electrochemical measurement system configured to electrochemically measure a specimen with using a measuring liquid, the electrochemical measurement system comprising an electrochemical measurement device (30, 30A, 60, 70, 80, 90, 100, 110) according to claim 11 and an electrochemical measurement apparatus,
wherein the electrochemical measurement apparatus includes:
a controller (41) that apples an electric potential to the working electrode (13);
a measurement unit (42) that detects whether the measuring liquid contacts the leakage detector (14) or not; and
a determination unit (43) that, when the measurement unit (42) detects that the measuring liquid contacts the leakage detector (14), determines that the measuring liquid in the first well (11) leaks.

20. The electrochemical measurement system according to claim 19,
wherein the measurement unit (42) includes an image sensor (150) that recognizes a change of the color of the discoloration portion, and
wherein, when the image sensor (150) detects the change of the color of the discoloration portion, the determination unit (43) determines that the measuring liquid in the first well (11) leaks.

## Patentansprüche

1. Elektrochemische Messvorrichtung, die zum elektrochemischen Messen einer Probe unter Verwendung einer Messflüssigkeit ausgeführt ist, wobei die elektrochemische Messvorrichtung umfasst:
eine erste Vertiefung (11), die so ausgeführt ist, dass die Probe in sie eingelegt wird und die Messflüssigkeit in sie eingespritzt wird;
eine Wand (7) mit einer Innenwandfläche (7A), die die erste Vertiefung (11) bildet, wobei die Wand (7) die erste Vertiefung (11) umgibt;
eine erste Arbeitselektrode (13), die mit der ersten Vertiefung (11) in Kontakt ist, wobei die erste Arbeitselektrode (13) so ausgeführt ist, dass ein elektrisches Potential an sie angelegt wird; und
einen Leck-Detektor (14), der ein Austreten der Messflüssigkeit erfasst,
**dadurch gekennzeichnet, dass** die erste Arbeitselektrode (13), die Wand (7) und der Leck-Detektor (14) so angeordnet sind, dass die erste Arbeitselektrode (13) von der Wand (7), die die erste Vertiefung (11) bildet, und von dem Leck-Detektor (14, 141, 142) umgeben ist, wobei ein unteres Ende der Innenwandfläche (7A) zwischen der ersten Arbeitselektrode (13) und dem Leck-Detektor (14) angeordnet ist,
und der Leck-Detektor (14) eine Leck-Erfassungselektrode (141) zum Erfassen eines Leckstroms enthält.

2. Elektrochemische Messvorrichtung nach Anspruch 1, wobei die Leck-Erfassungselektrode (141) die Innenwandfläche (7A) umgibt.

3. Elektrochemische Messvorrichtung nach Anspruch 2, wobei die Leck-Erfassungselektrode (141) eine Ringform hat.

4. Elektrochemische Messvorrichtung nach Anspruch 3, wobei die Leck-Erfassungselektrode (141) eine Ringform hat, die sich teilweise öffnet.

5. Elektrochemische Messvorrichtung nach Anspruch 1, wobei der Leckstrom in der Leck-Erfassungselektrode (141) fließt und die elektrochemische Messvorrichtung des Weiteren umfasst
einen Ableitabschnitt (141B) der Leck-Erfassungselektrode, der an einer Fläche angeordnet ist, an der die Leck-Erfassungselektrode (141) angeordnet ist, wobei der Ableitabschnitt (141B) der Leck-Erfassungselektrode über eine Verdrahtung (141C) mit der Leck-Erfassungselektrode (141) gekoppelt ist, und der Ableitabschnitt (141B) der Leck-Erfassungselektrode so ausgeführt ist, dass er den in der Leck-Erfassungselektrode (141) fließenden Leckstrom ableitet, und
wobei die Leck-Erfassungselektrode (141) zwischen der Innenwandfläche (7A) und dem Ableitabschnitt (141B) der Leck-Erfassungselektrode angeordnet ist.

6. Elektrochemische Messvorrichtung nach Anspruch 1,
wobei die Wand (17) aus einem Kunststoff besteht,
die Leck-Erfassungselektrode (141) in der Wand (17) angeordnet ist, und
eine Oberfläche der Leck-Erfassungselektrode (141) mit dem Kunststoff in Kontakt ist.

7. Elektrochemische Messvorrichtung nach Anspruch 1, die des Weiteren umfasst:
einen Auflege-Abschnitt (16), der in der ersten Vertiefung (11) angeordnet ist, wobei der Auflege-Abschnitt (16) so ausgeführt ist, dass die Probe auf ihn aufgelegt wird,
und die erste Arbeitselektrode (13) den Auflege-Abschnitt (16) umgibt.

8. Elektrochemische Messvorrichtung nach Anspruch 7, die des Weiteren eine zweite Arbeitselektrode (71) umfasst, die die erste Arbeitselektrode (13) umgibt.

9. Elektrochemische Messvorrichtung nach Anspruch 1, die des Weiteren eine Gegenelektrode (18, 18A, 18B, 81, 121) umfasst, die in der ersten Vertiefung (11) angeordnet ist, wobei die Gegenelektrode so ausgeführt ist, dass an sie ein elektrisches Potential angelegt wird.

10. Elektrochemische Messvorrichtung nach Anspruch 1, die des Weiteren umfasst:
eine zweite Vertiefung (11), die sich von der ersten Vertiefung (11) unterscheidet;
eine Gegenelektrode (18, 18A, 18B, 81, 121), die in der zweiten Vertiefung (11) angeordnet ist, wobei die Gegenelektrode so ausgeführt ist, dass an sie ein zur Messung verwendetes elektrisches Potential angelegt wird; und
einen Speicher (23), der oberhalb der ersten Vertiefung (11) und der zweiten Vertiefung (11) angeordnet ist, wobei der Speicher (23) so ausgeführt ist, dass er die Messflüssigkeit, die in die erste Vertiefung (11) und die zweite Vertiefung (11) eingespritzt werden soll, zusammen speichert.

11. Elektrochemische Messvorrichtung, die zum elektrochemischen Messen einer Probe unter Verwendung einer Messflüssigkeit ausgeführt ist, wobei die elektrochemische Messvorrichtung umfasst:
eine erste Vertiefung (11), die so ausgeführt ist, dass die Probe in sie eingelegt wird und die Messflüssigkeit in sie eingespritzt wird;
eine Wand (7) mit einer Innenwandfläche (7A), die die erste Vertiefung (11) bildet, wobei die Wand (7) die erste Vertiefung (11) umgibt;
eine erste Arbeitselektrode (13), die mit der ersten Vertiefung (11) in Kontakt kommt, wobei die erste Arbeitselektrode (13) so ausgeführt ist, dass ein elektrisches Potential an sie angelegt wird; und
einen Leck-Detektor (14), der ein Austreten der Messflüssigkeit erfasst,
**dadurch gekennzeichnet, dass** die erste Arbeitselektrode (13), die Wand (7) und der Leck-Detektor (14) so angeordnet sind, dass die erste Arbeitselektrode (13) von der Wand (7), die die erste Vertiefung (11) bildet, und von dem Leck-Detektor (14, 141, 142) umgeben ist, wobei ein unteres Ende der Innenwandfläche (7A) zwischen der ersten Arbeitselektrode (13) und dem Leck-Detektor (14) angeordnet ist,
und der Leck-Detektor (14) einen Entfärbungs-Abschnitt (142) zur Leck-Erfassung aufweist, dessen Farbe sich bei Kontakt mit der Messflüssigkeit ändert.

12. Elektrochemische Messvorrichtung nach Anspruch 11, wobei der Entfärbungs-Abschnitt (142) zur Leck-Erfassung ein mit einem wasserlöslichen Färbemittel beschichtetes Papierblatt ist.

13. Elektrochemisches Messsystem, das zum elektrochemischen Messen einer Probe unter Verwendung einer Messflüssigkeit ausgeführt ist, wobei das elektrochemische Messsystem eine elektrochemische Messvorrichtung (30, 30A, 60, 70, 80, 90, 100, 110) nach Anspruch 1 sowie eine elektrochemische Messeinrichtung umfasst,
und die elektrochemische Messeinrichtung enthält:
eine Steuerungseinrichtung (41), die ein elektrisches Potential an die Arbeitselektrode (13) anlegt;
eine Mess-Einheit (42), die erfasst, ob die Messflüssigkeit mit dem Leck-Detektor (14) in Kontakt ist; sowie
eine Feststellungs-Einheit (43), die, wenn die Mess-Einheit (42) erfasst, dass die Messflüssigkeit mit dem Leck-Detektor (14) in Kontakt ist, feststellt, dass die Messflüssigkeit in der ersten Vertiefung (11) austritt.

14. Elektrochemisches Messsystem nach Anspruch 13,
wobei, wenn die Leck-Erfassungselektrode (141) den Leckstrom erfasst, die Feststellungs-Einheit (43) feststellt, dass die Messflüssigkeit in der ersten Vertiefung (11) austritt.

15. Elektrochemisches Messsystem nach Anspruch 14,
wobei die elektrochemische Messvorrichtung des Weiteren eine Gegenelektrode (18) enthält, die in die erste Vertiefung (11) so eingeführt ist, dass sie mit der Messflüssigkeit in Kontakt ist, wobei die Gegenelektrode (18) so ausgeführt ist, dass an sie ein elektrisches Potential angelegt wird,
der Leckstrom zwischen der Gegenelektrode (18) und der Leck-Erfassungselektrode (141) fließt, und
die Mess-Einheit (42) den Leckstrom misst, der zwischen der Gegenelektrode (18) und der Leck-Erfassungselektrode (141) fließt.

16. Elektrochemisches Messsystem nach Anspruch 14,
wobei die elektrochemische Messvorrichtung des Weiteren eine Gegenelektrode (18) enthält, die in der ersten Vertiefung (11) angeordnet ist, und die Gegenelektrode (18) so ausgeführt ist, dass an sie ein elektrisches Potential angelegt wird,
der Leckstrom zwischen der Gegenelektrode (18) und der Leck-Erfassungselektrode (141) fließt, und
die Mess-Einheit (42) den Leckstrom misst, der zwischen der Gegenelektrode (18) und der Leck-Erfassungselektrode (141) fließt.

17. Elektrochemisches Messsystem nach Anspruch 14,
wobei die elektrochemische Messvorrichtung des Weiteren enthält:
eine zweite Vertiefung (11), die sich von der ersten Vertiefung (11) unterscheidet;
eine Gegenelektrode (18), die in der zweiten Vertiefung (11) angeordnet ist, wobei die Gegenelektrode (18) so ausgeführt ist, dass sie ein elektrisches Potential hat; sowie
einen Speicher (23), der oberhalb der ersten Vertiefung (11) und der zweiten Vertiefung (11) angeordnet ist, wobei der Speicher (23) so ausgeführt ist, dass er die Messflüssigkeit, die in die erste Vertiefung (11) und die zweite Vertiefung (11) eingespritzt werden soll, zusammen speichert; und
die Mess-Einheit (42) einen zwischen der Gegenelektrode (18) und der Leck-Erfassungselektrode (141) fließenden Strom misst.

18. Elektrochemisches Messsystem nach Anspruch 14,
wobei der Leckstrom zwischen der Arbeitselektrode (13) und der Leck-Erfassungselektrode (141) fließt, und
die Mess-Einheit (42) den zwischen der Arbeitselektrode (13) und der Leck-Erfassungselektrode (141) fließenden Leckstrom misst.

19. Elektrochemisches Messsystem, das zum elektrochemischen Messen einer Probe unter Verwendung einer Messflüssigkeit ausgeführt ist, wobei das elektrochemische Messsystem eine elektrochemische Messvorrichtung (30, 30A, 60, 70, 80, 90, 100, 110) nach Anspruch 11 sowie eine elektrochemische Messeinrichtung umfasst,
und die elektrochemische Messeinrichtung enthält:
eine Steuerungseinrichtung (41), die ein elektrisches Potential an die Arbeitselektrode (13) anlegt;
eine Mess-Einheit (42), die erfasst, ob die Messflüssigkeit mit dem Leck-Detektor (14) in Kontakt ist; sowie
eine Feststellungs-Einheit (43), die, wenn die Mess-Einheit (42) erfasst, dass die Messflüssigkeit mit dem Leck-Detektor (14) in Kontakt ist, feststellt, dass die Messflüssigkeit in der ersten Vertiefung (11) austritt.

20. Elektrochemisches Messsystem nach Anspruch 19,
wobei die Mess-Einheit (42) einen Bildsensor (150) enthält, der eine Änderung der Farbe des Entfärbungs-Abschnitts erkennt, und
wenn der Bildsensor (150) die Änderung der Farbe des Entfärbungs-Abschnitts erfasst, die Feststellungs-Einheit (43) feststellt, dass die Messflüssigkeit in der ersten Vertiefung (11) austritt.

## Revendications

1. Dispositif de mesure électrochimique configuré pour mesurer électrochimiquement un échantillon en utilisant un liquide de mesure, le dispositif de mesure électrochimique comprenant :
un premier puits (11) configuré pour avoir l'échantillon placé dans son intérieur et avoir le liquide de mesure injecté dans celui-ci ;
une paroi (7) ayant une surface de paroi interne (7A) formant le premier puits (11), la paroi (7) entourant le premier puits (11) ;
une première électrode de travail (13) en contact avec le premier puits (11), la première électrode de travail (13) étant configurée pour avoir un potentiel électrique appliquée à celle-ci ; et
un détecteur de fuite (14) qui détecte une fuite du liquide de mesure,
**caractérisé en ce que,** la première électrode de travail (13), la paroi (7) et le détecteur de fuite (14) sont agencés de telle sorte que la première électrode de travail (13) est entourée par la paroi (7) définissant le premier puits (11) et par le détecteur de fuite (14, 141, 142), une extrémité inférieure de la surface de paroi interne (7A) étant disposée entre la première électrode de travail (13) et le détecteur de fuite (14),
le détecteur de fuite (14) incluant une électrode de détection de fuite (141) pour détecter un courant de fuite.

2. Dispositif de mesure électrochimique selon la revendication 1, dans lequel l'électrode de détection de fuite (141) entoure la surface de paroi interne (7A).

3. Dispositif de mesure électrochimique selon la revendication 2, dans lequel l'électrode de détection de fuite (141) a une forme annulaire.

4. Dispositif de mesure électrochimique selon la revendication 3, dans lequel l'électrode de détection de fuite (141) a une forme annulaire s'ouvrant partiellement.

5. Dispositif de mesure électrochimique selon la revendication 1,
dans lequel le courant de fuite circule dans l'électrode de détection de fuite (141), le dispositif de mesure électrochimique comprenant en outre une partie d'extraction d'électrode de détection de fuite (141B) disposée sur une surface sur laquelle est disposée l'électrode de détection de fuite (141), la partie d'extraction d'électrode de détection de fuite (141B) étant couplée à l'électrode de détection de fuite (141) via un câblage (141C), la partie d'extraction d'électrode de détection de fuite (141B) étant configurée pour extraire le courant de fuite circulant dans l'électrode de détection de fuite (141), et
dans lequel l'électrode de détection de fuite (141) est disposée entre la surface de paroi interne (7A) et la partie d'extraction d'électrode de détection de fuite (141B).

6. Dispositif de mesure électrochimique selon la revendication 1,
dans lequel la paroi (17) est constituée d'une résine,
dans lequel l'électrode de détection de fuite (141) est disposée dans la paroi (17), et
dans lequel une surface de l'électrode de détection de fuite (141) est en contact avec la résine.

7. Dispositif de mesure électrochimique selon la revendication 1, comprenant en outre
une partie de placement (16) disposée dans le premier puits (11), la partie de placement (16) étant configurée pour avoir l'échantillon placé sur celle-ci,
la première électrode de travail (13) entourant la partie de placement (16).

8. Dispositif de mesure électrochimique selon la revendication 7, comprenant en outre une seconde électrode de travail (71) entourant la première électrode de travail (13).

9. Dispositif de mesure électrochimique selon la revendication 1, comprenant en outre une contre-électrode (18, 18A, 18B, 81, 121) disposée dans le premier puits (11), la contre-électrode étant configurée pour avoir un potentiel électrique appliquée à celle-ci.

10. Dispositif de mesure électrochimique selon la revendication 1, comprenant en outre :
un second puits (11) différent du premier puits (11) ;
une contre-électrode (18, 18A, 18B, 81, 121) disposée dans le second puits (11), la contre-électrode étant configurée pour avoir un potentiel électrique utilisé pour la mesure appliqué à celle-ci ; et
un réservoir (23) disposé au-dessus du premier puits (11) et du second puits (11), le réservoir (23) étant configuré pour tenir en réserve de façon commune le liquide de mesure à injecter dans le premier puits (11) et le second puits (11).

11. Dispositif de mesure électrochimique configuré pour mesurer électrochimiquement un échantillon en utilisant un liquide de mesure, le dispositif de mesure électrochimique comprenant :
un premier puits (11) configuré pour avoir l'échantillon placé dans son intérieur et avoir le liquide de mesure injecté dans celui-ci ;
une paroi (7) ayant une surface de paroi interne (7A) formant le premier puits (11), la paroi (7) entourant le premier puits (11) ;
une première électrode de travail (13) en contact avec le premier puits (11), la première électrode de travail (13) étant configurée pour avoir un potentiel électrique appliquée à celle-ci ; et
un détecteur de fuite (14) qui détecte une fuite du liquide de mesure,
**caractérisé en ce que,** la première électrode de travail (13), la paroi (7) et le détecteur de fuite (14) sont agencés de telle sorte que la première électrode de travail (13) est entourée par la paroi (7) définissant le premier puits (11) et par le détecteur de fuite (14, 141, 142), une extrémité inférieure de la surface de paroi interne (7A) étant disposée entre la première électrode de travail (13) et le détecteur de fuite (14),
le détecteur de fuite (14) comprenant une partie de décoloration de détection de fuite (142) présentant un changement de couleur au contact du liquide de mesure.

12. Dispositif de mesure électrochimique selon la revendication 11, la partie de décoloration de détection de fuite (142) étant une feuille de papier enduite d'un agent colorant hydrosoluble.

13. Système de mesure électrochimique configuré pour mesurer électrochimiquement un échantillon en utilisant un liquide de mesure, le système de mesure électrochimique comprenant un dispositif de mesure électrochimique (30, 30A, 60, 70, 80, 90, 100, 110) selon la revendication 1 et un appareil de mesure électrochimique,
l'appareil de mesure électrochimique incluant :
un module de commande (41) qui applique un potentiel électrique à l'électrode de travail (13) ;
une unité de mesure (42) qui détecte si le liquide de mesure est en contact avec le détecteur de fuite (14) ou non ; et
une unité de détermination (43) qui, lorsque l'unité de mesure (42) détecte que le liquide de mesure est en contact avec le détecteur de fuite (14), détermine que le liquide de mesure dans le premier puits (11) fuit.

14. Système de mesure électrochimique selon la revendication 13,
dans lequel, lorsque l'électrode de détection de fuite (141) détecte le courant de fuite, l'unité de détermination (43) détermine que le liquide de mesure dans le premier puits (11) fuit.

15. Système de mesure électrochimique selon la revendication 14,
dans lequel le dispositif de mesure électrochimique inclut en outre une contre-électrode (18) insérée dans le premier puits (11) de façon à être en contact avec le liquide de mesure, la contre-électrode (18) étant configurée pour avoir un potentiel électrique appliquée à celle-ci,
dans lequel le courant de fuite circule entre la contre-électrode (18) et l'électrode de détection de fuite (141), et
dans lequel l'unité de mesure (42) mesure le courant de fuite circulant entre la contre-électrode (18) et l'électrode de détection de fuite (141).

16. Système de mesure électrochimique selon la revendication 14,
dans lequel le dispositif de mesure électrochimique inclut en outre une contre-électrode (18) disposée dans le premier puits (11), la contre-électrode (18) étant configurée pour avoir un potentiel électrique appliqué à celle-ci,
dans lequel le courant de fuite circule entre la contre-électrode (18) et l'électrode de détection de fuite (141), et
dans lequel l'unité de mesure (42) mesure le courant de fuite circulant entre la contre-électrode (18) et l'électrode de détection de fuite (141).

17. Système de mesure électrochimique selon la revendication 14, le dispositif de mesure électrochimique incluant en outre :
un second puits (11) différent du premier puits (11) ;
une contre-électrode (18) disposée dans le second puits (11), la contre-électrode (18) étant configurée pour avoir un potentiel électrique ; et
un réservoir (23) disposé au-dessus du premier puits (11) et du second puits (11), le réservoir (23) étant configuré pour tenir en réserve de façon commune le liquide de mesure à injecter dans le premier puits (11) et le second puits (11), et
dans lequel l'unité de mesure (42) mesure un courant circulant entre la contre-électrode (18) et l'électrode de détection de fuite (141).

18. Système de mesure électrochimique selon la revendication 14,
dans lequel le courant de fuite circule entre l'électrode de travail (13) et l'électrode de détection de fuite (141), et
dans lequel l'unité de mesure (42) mesure le courant de fuite circulant entre l'électrode de travail (13) et l'électrode de détection de fuite (141).

19. Système de mesure électrochimique configuré pour mesurer électrochimiquement un échantillon en utilisant un liquide de mesure, le système de mesure électrochimique comprenant un dispositif de mesure électrochimique (30, 30A, 60, 70, 80, 90, 100, 110) selon la revendication 11 et un appareil de mesure électrochimique,
l'appareil de mesure électrochimique incluant :
un module de commande (41) qui applique un potentiel électrique à l'électrode de travail (13) ;
une unité de mesure (42) qui détecte si le liquide de mesure est en contact avec le détecteur de fuite (14) ou non ; et
une unité de détermination (43) qui, lorsque l'unité de mesure (42) détecte que le liquide de mesure est en contact avec le détecteur de fuite (14), détermine que le liquide de mesure dans le premier puits (11) fuit.

20. Système de mesure électrochimique selon la revendication 19,
dans lequel l'unité de mesure (42) inclut un capteur d'image (150) qui reconnaît un changement de couleur de la partie de décoloration, et
dans lequel, lorsque le capteur d'image (150) détecte le changement de couleur de la partie de décoloration, l'unité de détermination (43) détermine que le liquide de mesure dans le premier puits (11) fuit.
